# EUROPEAN PATENT APPLICATION

(11) **EP 1 561 456 A1**
(43) Date of publication of application: **10.08.2005**
(21) Application number: 03811135.7
(22) Date of filing: 13.11.2003
(51) Int. Cl.: A61K 7/32, A61K 7/38

(54) **COSMETIC COMPOSITIONS**

(30) Priority: 14.11.2002 JP 2002330610
(71) Applicant: Kao Corporation, Tokyo 103-8210 (JP)
(72) Inventor: NAMBU, Hiromi, c/o Kao Corporation, Wakayama-shi, Wakayama 640-8580 (JP); HAMAGUCHI, Takeshi, c/o Kao Corporation, Wakayama-shi, Wakayama 640-8580 (JP)
(74) Representative: HOFFMANN EITLE
(86) International application number: PCT/JP2003/014449
(87) International publication number: WO 2004/043421

(57) **Abstract**

The invention provides a cosmetic composition, particularly a cosmetic composition for antiperspiration. The invention relates to a cosmetic composition comprising surface-hydrophobated water-absorbing polymer particles and an anti-perspiring component, as well as a cosmetic composition comprising water-absorbing polymer particles, an anti-perspiring component, and at least one member selected from the group consisting of silicone having a viscosity (25°C) of not higher than 5 × 10⁻³ m²/s and hydrophobic particles, wherein the content of the water-absorbing polymer particles is 3 to 95 parts by weight relative to 100 parts by weight of the anti-perspiring component.

## Description

### Field of the invention

The present invention relates to a cosmetic composition useful for antiperspiration.

### Background of the invention

In perspiring sites particularly the armpit, stickiness due to perspiration worsens the skin feel and an unpleasant smell is generated, and thus cosmetics having an anti-perspiring effect have been used. Particularly, an aluminum compound is used as a main component exhibiting an anti-perspiring effect (JP-A 52-99236). However, sticky feel due to perspiration remains, and the anti-perspiring effect is not sufficient.

In order to reduce sticky feel due to perspiration, accordingly, cosmetics containing an anti-perspiring component and/or a sterilizing component and a highly water-absorbing polymer compound (JP-A 3-284617), and then cosmetics containing a novel aluminum compound as an anti-perspiring component have been known, but their anti-perspiring effect are not sufficient, and stickiness due to perspiration are often felt.

Further, when an antiperspirant based on an aluminum compound is applied onto the armpit and soap foam is applied onto the armpit at the time of shaving, the foam is broken thus causing not only a problem of deterioration in smoothness upon shaving with a razor, but also a problem that owing to components in the antiperspirant, soap scum is formed in a large amount in the armpit, to further deteriorate smoothness upon shaving with a razor.

Further, cosmetics containing hydrophobated powder and a water-absorbing polymer (JP-A 4-356415) are known in order to reduce sticky feel due to perspiration.

### Summary of the invention

The present invention relates to a cosmetic composition, particularly a cosmetic composition for antiperspiration.

The present invention provides a cosmetic composition containing surface-hydrophobated water-absorbing polymer particles and an anti-perspiring component.

The present invention provides use of a composition containing surface-hydrophobated water-absorbing polymer particles and an anti-perspiring component as cosmetics. The present invention also provides a method of antiperspiration, which includes applying a composition containing surface-hydrophobated water-absorbing polymer particles and an anti-perspiring component onto the skin.

### Detailed description of the invention

The present invention relates to a cosmetic composition having high anti-perspiring performance and excellent in smoothness at the time of shaving. The present invention provides a cosmetic composition preferably containing water-absorbing polymer particles, an anti-perspiring component, and at least one member selected from the group consisting of silicone having a viscosity (25°C) of not higher than 5 × 10⁻³ m²/s and hydrophobic particles, wherein the content of the water-absorbing polymer particles is 3 to 95 parts by weight relative to 100 parts by weight of the anti-perspiring component.

### [Water-absorbing polymer particles]

The shape of the water-absorbing polymer particles in the present invention is not particularly limited, and may be not only in the form of sphere, egg or lump, but also in the form of scale, plate, fiber, fine-powder agglomerate (granule) or in an amorphous state. Preferably, the water-absorbing polymer particles are in the form of sphere, egg or lump for easy availability, more preferably in the form of sphere for good feeling. These particles may be porous.

When the water-absorbing polymer particles used in the present invention are in the form of sphere, egg or lump, the average particle diameter thereof is preferably 0.1 µm or more, more preferably 0.5 µm or more, and the upper limit is preferably 50 µm or less, more preferably 20 µm or less, still more preferably 10 µm or less and even more preferably 5 µm or less, so that the resulting cosmetics are not remarkable as white powder, do not cause a sense of incongruity after absorption of water, have dry feel, and are not easily removed from the skin. The average particle diameter refers to the diameter of a particle not swollen with water, determined (in a cyclohexane solvent) with a light scattering particle-diameter measuring instrument (for example, LS-230 model manufactured by Coulter, Inc.).

The amount of water absorbed into the water-absorbing polymer particles used in the present invention is preferably not lower than 5 g/g, more preferably not lower than 10 g/g, to achieve sufficient anti-perspiring performance, and the upper limit is preferably not higher than 100 g/g, more preferably not higher than 50 g/g, still more preferably not higher than 30 g/g, in order to prevent the particles from giving slimy feel upon absorption of sweat on the skin and to make them hardly releasable from the skin. The amount of water absorbed can be determined by adding 1000 ml water to 5 g sample, then suspending and stirring it for 30 minutes (100 rpm, 25°C), centrifuging it at 2,000 G for 30 minutes, discarding the supernatant gently, measuring the weight of the sample, and determining the amount of water absorbed, on the basis of the difference of this weight from the initial weight of the sample.

As the polymer used in the water-absorbing polymer particles of the present invention, a natural polymer, a semi-synthetic polymer or a synthetic polymer can be used insofar as it is a polymer having the action of absorbing water. To attain a water-retaining property, it is preferably a polymer having a crosslinked structure, and such a polymer is a (co)polymer crosslinked by a crosslinking method described later or a (co)polymer having a crosslinkage via a hydrogen bond or hydrophobic bond, a crosslinkage derived from a partial crystalline structure or a crosslinkage derived from a helix structure etc. [(co)polymer means a polymer or copolymer.].

For example, the natural polymer and semi-synthetic polymer include starch, carrageenan, gelatin, agar, tragacanth gum, viscose, cellulose (for example, crystalline cellulose), methyl cellulose, ethyl cellulose, hydroxyethyl cellulose and carboxymethyl cellulose, or crosslinked products thereof, for example starch-(meth)acrylate graft copolymers (or its crosslinked products) [(meth)acrylate means acrylate, methacrylate or a mixture thereof.].

The synthetic polymer includes a crosslinked product of a (co)polymer of hydrophilic vinyl monomers such as anionic monomers or salts thereof, nonionic hydrophilic group-containing monomers, amino group-containing unsaturated monomers or neutralized products thereof or quaternarized products thereof. The hydrophilicity of the hydrophilic vinyl monomer means that the solubility in 100g of water (20°C) is preferably 6 weight% or more, more preferably more than 20 weight%. Examples of monomers used in production of the synthetic polymer include anionic monomers such as (meth) acrylic acid, maleic acid, fumaric acid, crotonic acid, itaconic acid, 2-(meth)acryloyl ethane sulfonic acid, 2-(meth)acryloyl propane sulfonic acid, 2-(meth)acrylamide-2-methyl propane sulfonic acid, vinyl sulfonic acid and styrene sulfonic acid or salts thereof; nonionic hydrophilic group-containing monomers such as (meth)acrylamide, N-substituted (meth)acrylamide, 2-hydroxyethyl (meth)acrylate, 2-hydroxypropyl (meth)acrylate, methoxypolyethylene glycol (meth)acrylate, polyethylene glycol(meth)acrylate, N-vinyl pyrrolidone and N-vinyl acetamide; and amino-group containing unsaturated monomers such as N,N-dimethylaminoethyl (meth)acrylate, N,N-dimethylaminopropyl (meth)acrylate and N, N-dimethylaminopropyl (meth) acrylamide or acid-neutralized product thereof, or quaternized products thereof. Preferable examples of the acid used in producing the acid-neutralized product include hydrochloric acid, sulfuric acid, nitric acid, acetic acid, formic acid, maleic acid, fumaric acid, citric acid, tartaric acid, adipic acid, lactic acid etc., and preferable examples of the quaternizing agent include alkyl halides such as methyl chloride, ethyl chloride, methyl bromide and methyl iodide, and standard alkylating agents such as dimethyl sulfate, diethyl sulfate and di-n-propyl sulfate. The counterion includes halogen ions such as chlorine and organic anions such as methosulfate. Unless the hydrophilicity of the resultant polymer is extremely inhibited, the hydrophobic vinyl monomers such as acrylates and styrene or derivatives thereof can also be simultaneously copolymerized in an amount of preferably 0 to 50% by weight, more preferably 0 to 20 weight%, of the total monomers.

The hydrophobicity of the hydrophobic vinyl monomer means that the solubility in 100g of water (20°C) is preferably less than 6 weight%. No lower limit is provided. It may be 0.01 weight% or more.

As the monomer components used, one or more of those enumerated above can be selected, but crosslinked (co)polymers of α,β-unsaturated carboxylic acid monomers such as (meth) acrylic acid, maleic acid, fumaric acid, crotonic acid, itaconic acid or salts thereof are preferable because of their high water-absorbing ability and easy availability. In addition to the α,β-unsaturated carboxylic acid monomers, other monomers can also be copolymerized.

To improve the water-absorbing ability, the amount of the hydrophilic vinyl monomers blended is preferably at least 50% by weight, more preferably at least 70% by weight and particularly preferably at least 90% by weight of the total monomers constituting the water-absorbing polymer particles.

The water-absorbing polymer particles are preferably a crosslinked polymer or copolymer of hydrophilic vinyl monomers and/or salt thereof, more preferably a crosslinked polymer or copolymer of α,β-unsaturated carboxylic acid monomers and/or salts thereof, particularly preferably crosslinked poly(meth)acrylate. These water-absorbing polymers can be used alone or in combination thereof.

The "salt" includes e.g. alkali metal salts (sodium salt, potassium salt, lithium salt etc.), alkaline earth metal salts (calcium salt, magnesium salt, barium salt etc.) and ammonium salts (quaternary ammonium salt, quaternary alkyl ammonium salt etc.). Among these, the sodium salt is even more inexpensive and preferable. Here, the degree of neutralization of the water-absorbing polymer particles is preferably 0.01 to 100%, more preferably 1 to 99% and particularly preferably 40 to 95% based on the number of moles of the acid group in the water-absorbing polymer. In the present invention, the "degree of neutralization" refers to the ratio (on a molar basis) of the salt-forming acid group to the acid group in the water-absorbing polymer, that is, (number of moles of the salt-forming acid group)/(number of moles of the salt-formable free acid plus the salt-forming acid group) × 100 (%).

The method of forming the crosslinked (co)polymers described above involves covalent crosslinking by (a) self-crosslinking during polymerization, (b) copolymerization with a multifunctional monomer, and (c) irradiation with radiation, or ionic crosslinking via (d) polyvalent metal ion. Among these, (b) is preferable from the viewpoint of easy production and stability of the crosslinked structure, and the multifunctional monomer includes a crosslinking vinyl monomer having at least 2 reactive unsaturated groups in the molecule and a compound having at least 2 functional groups other than unsaturated groups in the molecule (the above crosslinking vinyl monomer and the above compound are referred to collectively as crosslinking agent) .

Among these crosslinking vinyl monomers having at least 2 reactive unsaturated groups in the molecule, ethylene glycol di(meth)acrylate, polyethylene glycol di(meth)acrylate, divinyl benzene, pentaerythritol triallyl ether, pentaerythritol tetraallyl ether, and methylene bisacrylamide are preferable.

The compound having at least 2 functional groups other than unsaturated groups in the molecule is more preferably ethylene glycol diglycidyl ether or polyethylene glycol diglycidyl ether.

The amount of the crosslinking agent added is varied depending on the type of the crosslinking agent and the crosslinking method, but is preferably 0.001 part by weight or more, more preferably 0.01 part by weight or more, particularly preferably 0.1 part by weight or more, relative to 100 parts by weight of the total monomers constituting the water-absorbing polymer particles, and the upper limit is preferably 20 parts by weight or less, more preferably 10 parts by weight or less, particularly preferably 5 parts by weight or less. When the amount of the crosslinking agent is 0.001 part by weight or more, the amount of water absorbed can be maintained due to a lower content of water-soluble components in the resulting water-absorbing polymer, while when its amount is 20 parts by weight or less, the density of crosslinkages is suitable and the amount of water absorbed into the resultant water-absorbing polymer is sufficient.

The water-absorbing polymer particles used in the present invention are preferably water-absorbing polymer particles wherein at least a part of the surfaces of water-absorbing polymer particles was hydrophobated (referred to hereinafter as surface-hydrophobated water-absorbing polymer particles).

Such surface-hydrophobated water-absorbing polymer particles include, for example, (1) surface-hydrophobated water-absorbing polymer particles wherein the surfaces of water-absorbing polymer particles were coated with a silicone compound having at least one kind of functional group, (2) surface-hydrophobated water-absorbing polymer particles wherein the surfaces of water-absorbing polymer particles were coated with a polymer of a hydrophobic vinyl monomer, (3) surface-hydrophobated water-absorbing polymer particles wherein the surfaces of water-absorbing polymer particles were treated with a fluorine-based surfactant, and (4) surface-hydrophobated water-absorbing polymer particles wherein the surfaces of water-absorbing polymer particles were treated with a silane coupling agent. Among these polymer particles, the surface-hydrophobated water-absorbing polymer particles wherein the surfaces of water-absorbing polymer particles were coated with a silicone compound having at least one kind of functional group are preferable. Hereinafter, preferable surface-hydrophobated water-absorbing polymer particles are specifically described.

### (1) Surface-hydrophobated water-absorbing polymer particles wherein the surfaces of water-absorbing polymer particles were coated with a silicone compound having at least one kind of functional group

The silicone compound having at least one kind of functional group is a silicone compound having at least 2 silicon atoms, having at least one kind of functional group capable of chemical bonding, preferably covalent bonding and/or ionic bonding, to the surfaces of water-absorbing polymer particles.

In the surface-hydrophobated water-absorbing polymer particles, the surfaces of the water-absorbing polymer particles have been coated preferably via chemical bonding with a silicone compound having at least one kind of functional group. The silicone compound can thereby be stably present on the surfaces of the water-absorbing polymer particles, even upon incorporation into cosmetics. The presence of such chemical bonding can be confirmed by the fact that the silicone compound is present on the water-absorbing polymer particles even if the silicon-modified polymer particles (10% by weight relative to chloroform) are subjected 3 times to a washing step of treating with chloroform for 2 hours under stirring (30 rpm, 50°C) and then centrifugal separation.

The water-absorbing polymer particles are preferably coated thereon with the silicone compound to such an extent that the stickiness of the surfaces of the water-absorbing swollen particles can be suppressed.

Assuming that the amount of the total polymer particles (including the silicone compound) is 100 parts by weight, the lower limit of the amount of the silicone compound having at least one kind of functional group in the water-absorbing polymer particles is preferably 0.1 part by weight or more, more preferably 0.5 part by weight or more, still more preferably 1 part by weight or more. The upper limit is preferably 30 parts by weight or less, more preferably 10 parts by weight or less, still more preferably 5 parts by weight or less.

To prevent gel blocking among polymer particles or sticky feeling during use, the silicone compound having at least one kind of functional group is preferably hydrophobic. In particular, when the silicone compound is the one having plural kinds of functional groups and having a functional group not contributing to the reaction, the silicone compound is preferably hydrophobic.

As used herein, the hydrophobic silicone compound having at least one kind of functional group refers to the one whose solubility in 100 g water at 25°C is not more than 10% by weight, preferably not more than 1% by weight, more preferably not more than 0.5% by weight, even more preferably not more than 0.1% by weight. The lower limit of the solubility is not particularly present, but may be not less than 0.01% by weight.

The weight average molecular weight of the silicone compound having at least one kind of functional group is preferably 1000 to 500,000, more preferably 3000 to 200,000 and particularly preferably 10,000 to 200,000. Measurement of this weight average molecular weight is conducted by gel permeation chromatography (GPC), using polystyrene as standard and chloroform as eluent.

The functional group is preferably at least one kind of functional group selected from the group consisting of an amino group, an ammonium group, a carboxy group, a hydroxy group, an epoxy group and a radical-polymerizable unsaturated group, more preferably an amino group and/or an ammonium group. These groups may be located at a side chain, one end and/or both ends of siloxane, or may be a mixture thereof.

Typical examples of the silicone compound having at least one kind of functional group used in the present invention are shown below.

### (1-1) Silicone compound having an amino group and/or an ammonium group (referred to hereinafter as amino-modified silicone)

The amino-modified silicone is preferably the one having a polymerizable unit represented by the following general formula (I): 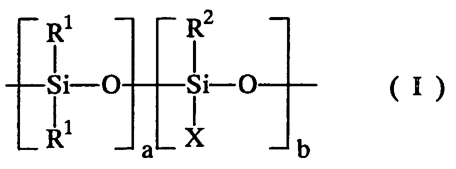 wherein R¹ represents a hydrogen atom or a C₁₋₆ hydrocarbon group, and a plurality of R¹s may be the same or different; R² represents R¹ or X whereupon X is a reactive functional group represented by -R³-Z (R³ represents a direct bond or a C₁₋₂₀ divalent hydrocarbon group, and Z represents a primary to tertiary amino group-containing group or a quaternary ammonium group-containing group); and a is a number of 2 or more, and b is a number of 1 or more.

In the general formula (I), R¹ groups independently represent a hydrogen atom or a C₁₋₆ hydrocarbon group, for example an alkyl group or phenyl group, preferably a methyl group or ethyl group, more preferably a methyl group. R³ is preferably a C₁₋₆ linear or branched alkylene group, and includes a methylene group, ethylene group, trimethylene group, propylene group, tetramethylene group etc., more preferably a trimethylene group or propylene group. a and b each represent the number of polymerizing repeating units.
Preferably, a is a number of 2 to 1000, and b is a number of 1 to 50. Z is preferably an amino group- or ammonium group-containing group represented by the general formula (II) or (III) below. 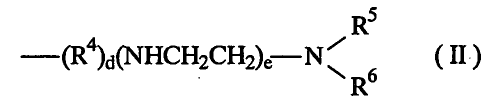 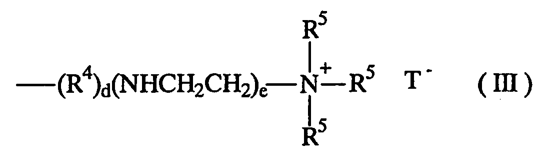 wherein R⁴ represents ―OCH₂CH₂―, 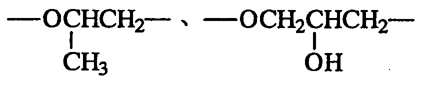 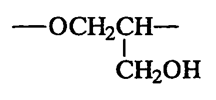 R⁵ and R⁶ each represent a hydrogen atom or a monovalent hydrocarbon group, d and e each represent an integer of 0 to 6, and T⁻ represents a halogen atom or an organic anion.

In the general formula (III), a plurality of R⁵s may be the same or different. Specific examples of T- include halogen ions such as chlorine, iodine, bromine etc. and organic anions such as methosulfate, ethosulfate, methophosphate, ethophosphate etc.

In the general formula (I), the group X is preferably -(CH₂)₃-NH₂, -(CH₂)₃-N(CH₃)₂-, -(CH₂)₃-NH-(CH₂)₂-NH₂, -(CH₂)₃-NH-(CH₂)₂-N(CH₃)₂, or -(CH₂)₃-N⁺(CH₃)₃Cl⁻, more preferably - (CH₂)₃-NH-(CH₂)₂-NH₂.

The weight average molecular weight of the amino-modified silicone is preferably 3000 to 200,000, and for easy reaction with anionic functional groups of the water-absorbing polymer and for hydrophobicity of the silicone compound, the amine equivalent thereof is preferably 250 to 10000 g/mol, more preferably 1000 to 5000 g/mol. That is, the amount of the amino group or ammonium group in the polymer is preferably 0.1 to 4 mmol/g, more preferably 0.2 to 1 mmol/g. The amine equivalent can be measured in a solvent such as ethanol by titration with hydrochloric acid of known concentration.

### (1-2) Silicon compound having a carboxy group (referred to hereinafter as carboxy-modified silicone)

The carboxy-modified silicone is preferably either a compound having a silicon atom and carboxy group bound via a saturated hydrocarbon, or an organosiloxane compound having a carboxyl group and/or a salt thereof bound to a silicon atom via a structure shown in one of the formulae (IV) and (V), which is described in JP-A 2002-114849: 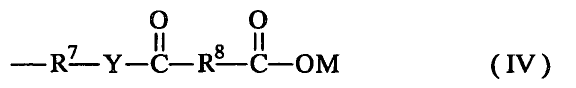 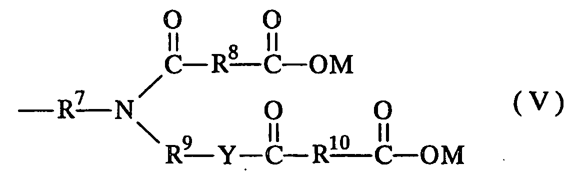 wherein R⁷, R⁸, R⁹ and R¹⁰ are the same or different and each represent a C₂₋₂₂ linear or branched alkylene or alkenylene group or an arylene group, which may have a substituent group containing a heteroatom; Y represents an -O- or -NH- group; and M represents a hydrogen atom, a metal, ammonium, C₁₋₂₂ alkyl or alkenyl ammonium, C₁₋₂₂ alkyl or alkenyl-substituted pyridinium, C₁₋₂₂ alkanol ammonium, or a basic amino acid.

Further, an amphoteric ionomer siloxane having two functional groups i.e. a carboxyl group and an ammonium group described in JP-A 6-1711 can also be preferably used.

The weight average molecular weight of the carboxy-modified silicone is preferably 3000 to 200,000. For easy reaction with cationic functional groups of the water-absorbing polymer and for hydrophobicity of the silicone compound, the carboxy equivalent thereof is preferably 250 to 10000 g/mol, more preferably 1000 to 5000 g/mol. That is, the amount of the carboxy group in the polymer is preferably 0.1 to 4 mmol/g, more preferably 0.2 to 1 mmol/g. The carboxy equivalent can be measured in a solvent such as ethanol by titration with NaOH of known concentration.

### (1-3) Silicone compound having a hydroxyl group (referred to hereinafter as hydroxyl-modified silicone)

The hydroxy-modified silicone includes the branched silicone of the general formula (VI), the both-terminal type silicone of the general formula (VII), the one-terminal type silicone of the general formula (VIII), and an alkylglyceryl ether-modified silicone disclosed in JP-A 5-112424. 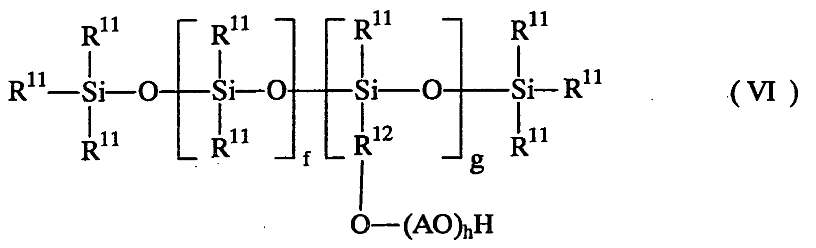 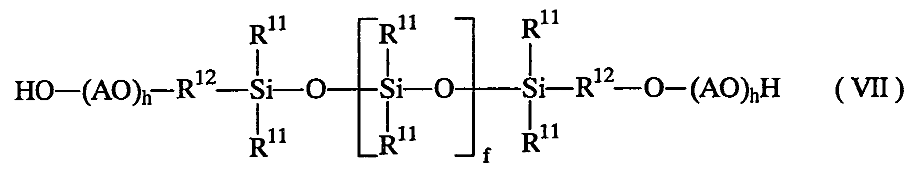 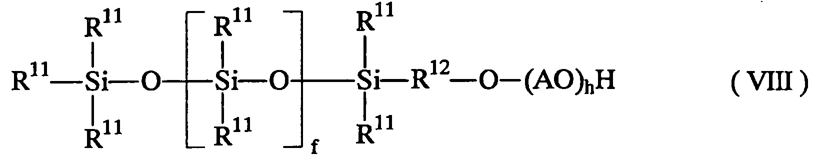 wherein R¹¹ groups may be the same or different and each represent a C₁₋₃ alkyl group, preferably a methyl group; R¹² groups may be the same or different and each represent a C₁₋₈ alkylene group, preferably a trimethylene group; AO groups may be the same or different and each represent an ethyleneoxy group or propyleneoxy group; f and g each are an integer of 1 or more; and h is an integer of 0 or 1 or more.

### (1-4) Silicone compound having an epoxy group (referred to hereinafter as epoxy-modified silicone)

The epoxy-modified silicone is preferably a silicone compound containing an epoxy group at one and/or both ends represented by the general formula (IX): 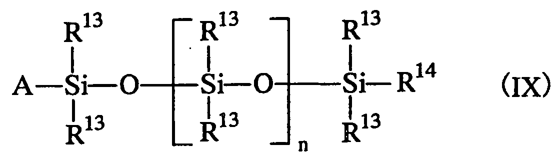 wherein R¹³ groups are the same or different and each represent a monovalent hydrocarbon group; R¹⁴ represents an epoxy-containing group or a monovalent hydrocarbon group; A represents an epoxy-containing group; and n is a number of 1 to 10000.

The monovalent hydrocarbon group represented by R¹³ includes an alkyl group such as methyl group and ethyl group, a cycloalkyl group such as cyclohexyl group, an aryl group such as phenyl group, and a fluorine atom-substituted alkyl group such as trifluoropropyl group. All R¹³ groups may be the same or different, but all R¹³ groups are desirably methyl groups.

In the general formula (IX), the epoxy-containing group represented by A is not particularly limited, and specific examples include the groups shown in the general formulae (X) to (XII) : 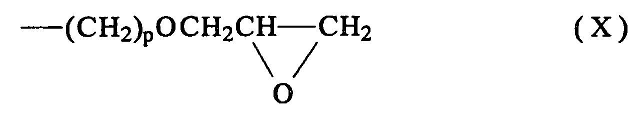 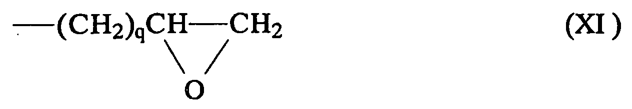 wherein p and q each are an integer of 1 or more. 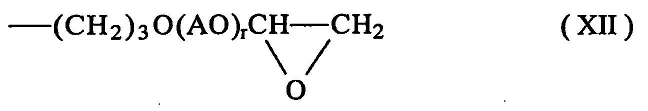 wherein r is an integer of 1 or more, and AO groups whose number is r are the same or different and each represent an ethyleneoxy group or propyleneoxy group.

In the general formula (IX), when R¹⁴ represents the same group as in R¹³, the compound is silicone containing an epoxy group at one end, while when R¹⁴ represents the same group as in A, the compound is silicone containing an epoxy group at both ends.

The epoxy-modified silicone is preferably silicone containing an epoxy group at one end, that is, silicone wherein R¹⁴ is the same group as in R¹³, wherein the hydrocarbon group is preferably an alkyl group such as methyl group and ethyl group, a cycloalkyl group such as cyclohexyl group, an aryl group such as phenyl group, and a fluorine atom-substituted alkyl group such as trifluoropropyl group, more preferably a methyl group.

In the general formula (IX), n is preferably 1 to 500, more preferably 5 to 100 and particularly preferably 10 to 50.

### (1-5) Silicone compound having a radical polymerizable unsaturated group

Preferable examples of the silicone compound having a radical polymerizable unsaturated group include a polysiloxane compound having a radical polymerizable group at one end described in JP-A 11-181003.

### (2) Surface-hydrophobated water-absorbing polymer particles wherein the surfaces of water-absorbing polymer particles were coated with a polymer of a hydrophobic vinyl monomer

The surface-hydrophobated water-absorbing polymer particles are water-absorbing polymer particles wherein the surfaces of water-absorbing polymer particles were coated with a polymer of at least one kind of hydrophobic vinyl monomer.

At least a part of the surfaces of the water-absorbing polymer particles is coated to such an extent as to suppress the stickiness of the surfaces of the water-absorbing swollen particles.

The lower limit of the amount of the polymer of hydrophobic vinyl monomers in the surface-hydrophobated water-absorbing polymer particles is preferably 1 weight part or more, more preferably 5 weight parts or more, particularly preferably 10 weight parts or more, relative to 100 parts by weight of the water-absorbing polymer particles. The upper limit is preferably 1000 weight parts or less, more preferably 400 weight parts or less, particularly preferably 200 weight parts or less. This range is preferable because there is no sticky feeling after absorption of water, and the speed of absorption of water is not particularly inhibited.

The hydrophobic vinyl monomer used in the present invention is a polymerizable monomer giving a hydrophobic polymer by polymerization, and can be polymerized by a polymerization method with a general radical polymerization initiator or irradiation with ultraviolet light.

The hydrophobic vinyl monomer is a monomer having the hydrophobicity defined above, and a usual radical polymerizable hydrophobic vinyl monomer is preferably used. Examples of the monomer include, for example, styrene, acrylates such as vinyl acetate, divinyl benzene, butyl acrylate, 2-ethylhexyl acrylate, cyclohexyl acrylate, decyl acrylate, lauryl acrylate, dodecenyl acrylate, myristyl acrylate, palmityl acrylate, hexadecenyl acrylate, stearyl acrylate, octadecenyl acrylate, behenyl acrylate etc., methacrylates such as butyl methacrylate, 2-ethylhexyl methacrylate, cyclohexyl methacrylate, decyl methacrylate, lauryl methacrylate, dodecenyl methacrylate, myristyl methacrylate, palmityl methacrylate, hexadecenyl methacrylate, stearyl methacrylate, octadecenyl methacrylate, behenyl methacrylate etc., fluorine-based monomers such as trifluoroethyl methacrylate, silicone macromonomers etc. These hydrophobic monomers can be used as a mixture of one or more thereof. Among these monomers, styrene and alkyl (meth)acrylates having a C₁₋₂₂ alkyl group are highly hydrophobic, easily available and preferable.

The weight average molecular weight of the hydrophobic polymer used in the present invention is preferably 1000 to 500,000, more preferably 3000 to 200,000 and particularly preferably 10,000 to 200,000. Measurement of this weight average molecular weight is conducted by gel permeation chromatography (GPC), using polystyrene as standard. The column used was Shodex KF-806 manufactured by Showa Denko K.K.

To improve the adhesion of the formed polymer to the water-adsorbing polymer, the hydrophilic vinyl monomer may be copolymerized to such an extent that the hydrophobicity of the resulting polymer is not extremely inhibited.

Such hydrophilic vinyl monomer includes α,β-unsaturated carboxylic acids such as (meth)acrylic acid, maleic acid, itaconic acid etc., maleic anhydride, chloromethyl styrene, glycidyl (meth)acrylate, (meth)acryloyloxyethyl isocyanate, 3-(trimethoxysilyl)propyl (meth)acrylate, dimethylaminoethyl (meth)acrylate, hydroxyethyl (meth)acrylate, vinyl pyridine, (meth)acrylamide etc. These hydrophilic vinyl monomers can be used as a mixture of two or more thereof. The amount of the hydrophilic vinyl monomer used is preferably 0 to 50 weight%, more preferably 0 to 20 weight%, of the total monomers constituting the polymer of the hydrophobic vinyl monomer.

### (3) Surface-hydrophobated water-absorbing polymer particles wherein the surfaces of water-absorbing polymer particles were treated with a fluorine-based surfactant

A hydrophilic moiety of the fluorine-based surfactant can be any of four kinds of moieties, that is, anionic, nonionic, cationic and amphoteric, while a hydrophobic moiety of the surfactant can make use of a fluorocarbon chain or a perfluorocarbon chain. The fluorine-based surfactant preferably has a functional group capable of reacting with the surfaces of the water-absorbing particles, and the hydrophobic moiety is preferably anionic, cationic or amphoteric, more preferably cationic or amphoteric. The carbon chain in the hydrophobic moiety may be a linear or branched chain. For example, the following fluorine-based surfactants can be mentioned.

Fluoroalkyl (C₂ to C₁₀) carboxylic acids, disodium N-perfluorooctane sulfonyl glutamate, sodium 3- [fluoroalkyl (C₆ to C₁₁)oxy]-1-alkyl (C₃ to C₄) sulfonate, sodium 3-[ω-fluoroalkanoyl (C₆ to C₈)-N-ethylamino]-1-propane sulfonate, N-[3-(perfluorooctanesulfonamide) propyl]-N,N-dimethyl-N-carboxymethylene ammonium betaine, fluoroalkyl (C₁₁ to C₂₀) carboxylic acid, perfluoroalkyl carboxylic acid (C₇ to C₁₃), perfluorooctane sulfonic acid diethanol amide, perfluoroalkyl (C₄ to C₁₂) sulfonates (Li, K, Na), N-propyl-N- (2-hydroxyethyl) perfluorooctane sulfonamide, perfluoroalkyl (C₆ to C₁₀) sulfonamide propyl trimethyl ammonium salt, perfluoroalkyl (C₆ to C₁₀)-N-ethyl sulfonyl glycine salt (K), bis(N-perfluorooctylsulfonyl-N-ethylaminoethyl) phosphate, monoperfluoroalkyl (C₆ to C₁₆) ethyl phosphate, perfluoroalkyl quaternary ammonium iodide (trade name: Florade FC-135, a cationic fluorine-based surfactant manufactured by Sumitomo 3M Ltd.), perfluoroalkyl alkoxylate (trade name: Florade FC-171, a nonionic fluorine-based surfactant manufactured by Sumitomo 3M Ltd.) and potassium perfluoroalkyl sulfonates (trade names: Florade FC-95 and FC-98, anionic surfactants manufactured by Sumitomo 3M Ltd.).

### (4) Surface-hydrophobated water-absorbing polymer particles wherein the surfaces of water-absorbing polymer particles were treated with a silane coupling agent

As the silane coupling agent, a silane coupling described in JP-A 61-211305 can be used.

Preferable examples include γ-glycidoxy propyl trimethoxy silane, γ-glycidoxy propyl methyl diethoxy silane, γ-(2-aminoethyl)aminopropyl trimethoxy silane, γ-(2-aminoethyl) aminopropyl methyl diethoxy silane, γ-aminopropyl triemethoxy silane, γ-chloropropyl trimethoxy silane, γ-chloropropyl methyl dimethoxy silane etc.

### [Method of producing water-absorbing polymer particles]

### (1) Method of producing water-absorbing polymer particles by polymerizing a hydrophilic vinyl monomer

When a polymer of a hydrophilic vinyl monomer is used in the water-absorbing polymer, the hydrophilic vinyl monomer and crosslinking agent may be polymerized by any method, but a method of polymerizing an aqueous solution of the hydrophilic vinyl monomer (preferably at a concentration of 1 to 70% by weight) is preferable, and various methods such as aqueous solution polymerization, reverse phase suspension polymerization and pearl polymerization can be used. In particular, aqueous solution polymerization or reverse phase suspension polymerization is preferable in respect of operativeness for polymerization and the water-absorbing performance of the resultant water-absorbing polymer, and reverse phase suspension polymerization is particularly preferable in respect of higher water-absorbing performance of the water-absorbing polymer. The temperature for polymerization of the hydrophilic vinyl monomer is preferably 20 to 120°C and the polymerization time is preferably 20 to 180 minutes.

As the polymerization initiator, a water-soluble radical polymerization initiator, for example a peroxide, hydroperoxide or an azo compound is known in a known amount. These polymerization initiators can be used as a mixture of at least two kinds thereof, or can be used as redox type polymerization initiators by adding chromium ion, sulfite, hydroxylamine, hydrazine etc. thereto. If necessary, an oil-soluble radical polymerization initiator, for example a peroxide type initiator such as benzoyl peroxide, lauroyl peroxide, tert-butyl peroxy pivalate or diisopropyl peroxy dicarbonate, or an azo type initiator such as azobis(isobutyronitrile), azobis(2,4-dimethylvaleronitrile), azobis(dimethyl isobutyrate) or azobis(cyclohexane carbonitrile) can also be used.

The amount of the water-soluble polymerization initiator is preferably 0.03 to 5 weight%, more preferably 0.1 to 2 weight%, based on the amount of the hydrophilic vinyl monomer.

A dispersant is used to disperse and stabilize the hydrophilic vinyl monomer stably in the oil phase (solvent). The dispersant includes a general anionic, cationic, nonionic or amphoteric surfactant or a natural, semi-synthetic or synthetic polymer. Examples thereof include an anionic surfactant such as sodium polyoxyethylene dodecyl ether sulfate and sodium dodecylether sulfate, a cationic or amphoteric surfactant such as trimethylstearylammonium chloride and carboxymethyldimethylcetyl ammonium, a sucrose fatty ester such as sucrose monostearate and sucrose dilaurate, a sorbitan ester such as sorbitan monostearate, a nonionic surfactant such as polyoxyalkylene adducts of sorbitan esters, such as polyoxyethylenesorbitan monostearate, a natural or semi-synthetic polymer such as cellulose derivatives such as starch or derivatives thereof, cellulose ethers such as ethyl cellulose and cellulose esters such as cellulose acetate, and a synthetic polymer such as polyvinyl alcohol or derivatives thereof, maleic group-containing polybutadiene and a quaternary salt of styrene-dimethylaminoethyl methacrylate.

To produce the surface-hydrophobated water-absorbing polymer particles, the silicone compound having at least one kind of functional group or the fluorine-based surfactant is preferably used as the dispersant. It may be combined with the other dispersant described above. Among the silicone compounds, the dispersant is preferably a silicone compound having at least one kind of functional group selected from the group consisting of an amino group, an ammonium group, a hydroxy group and a carboxy group.

The dispersant is present in an amount of preferably 0.5 to 30 parts by weight, more preferably 1 to 10 parts by weight, still more preferably 1 to 7 parts by weight, relative to 100 parts by weight of the total vinyl monomers constituting the water-absorbing polymer particles.

The solvent used in reverse phase suspension polymerization is preferably a hydrocarbon type solvent or silicone type solvent or a mixture thereof. Examples of the hydrocarbon type solvent include aliphatic hydrocarbons such ashexane, heptane, dodecane, cyclohexane, methylcyclohexane, isooctane and hydrogenated triisobutylene and aromatic hydrocarbons such as benzene, toluene, xylene and ethyl benzene, and examples of the silicone type solvent include octamethyl cyclotetrasiloxane, decamethyl cyclopentasiloxane, hexamethyl disiloxane and octamethyl trisiloxane. Among these solvents, hexane and cyclohexane are particularly preferable.

Before water or solvent in the reaction system is distilled away after polymerization, the surface of particle may be crosslinked by adding a crosslinking agent later and reacting it by heating preferably at 40 to 150°C.

### (2) Method of producing surface-hydrophobated water-absorbing polymer particles from water-absorbing polymer particles

The surface-hydrophobated water-absorbing polymer particles can be produced from water-absorbing polymer particles after polymerization or from previously obtained water-absorbing polymer particles such as a naturally occurring polymer.

The method is preferably a production method which includes heating water-absorbing particles and a silicone compound having at least one kind of functional group in the presence of a fluorine-based surfactant or a silane coupling agent, in the presence of water preferably a solvent, to which a crosslinking agent and/or a radical polymerization initiator is added if necessary. By the presence of water, functional groups in the water-absorbing polymer particles are dissociated to react readily with functional groups in the silicone compound having at least one kind of functional group, in the fluorine-based surfactant or in the silane coupling agent. The amount of the silicone compound having at least one kind of functional group, the fluorine-based surfactant or the silane coupling agent is preferably 0. 001 part by weight or more, more preferably 0.01 part by weight or more, particularly preferably 0.1 part by weight or more, relative to 100 parts by weight of the water-absorbing polymer (particles), and the upper limit is preferably 20 parts by weight or less, more preferably 10 parts by weight or less, particularly preferably 5 parts by weight or less.

As the solvent, the above-mentioned hydrocarbon type solvent or silicone type solvent or a mixture thereof is preferably used. The amount of water in the reaction system is preferably 1 to 200 parts by weight, more preferably 10 to 100 parts by weight, relative to 100 parts by weight of the water-absorbing polymer (particles). After the reaction, the water is removed, for example, by distillation. When the crosslinking agent is used, the crosslinking agent is allowed to be present in an amount of preferably 0.01 to 10 parts by weight, more preferably 0.01 to 5 parts by weight, still more preferably 0.01 to 3 parts by weight, relative to 100 parts by weight of the water-absorbing polymer (particles).

When the silicone compound having a radically polymerizable unsaturated group is used, the above-mentioned oil-soluble radical polymerization initiator is preferably allowed to be coexistent. The silicone compound having at least one kind of functional group or the fluorine-based surfactant can be added directly or as a solution wherein the silicone compound or the fluorine-based surfactant has been solubilized or dispersed by emulsification in an organic solvent, a surfactant or a dispersant, or in the form of a spray depending on the case. Heating for facilitating surface treatment is preferably in the range of 40 to 150°C.

The silicone compound having at least one kind of functional group, the fluorine-based surfactant or the silane coupling agent may be mixed with the water-absorbing polymer before or after disruption of the polymer, but it is more preferable that the water-absorbing polymer is disrupted to form water-absorbing polymer particles whose water content is regulated in a suitable range by drying if necessary, and the water-absorbing polymer particles are mixed, in e.g. a kneader, with them.

The water-absorbing polymer particles coated with the polymer of the hydrophobic vinyl monomer is preferably the one obtained by adding the hydrophobic vinyl monomer and the above-mentioned oil-soluble polymerization initiator to a reverse phase suspension polymerization solution during or after polymerization of the water-absorbing polymer particles, that is, slurry having the water-absorbing polymer particles dispersed in a solvent, followed by polymerizing the monomer. The solvent is preferably the above-mentioned hydrocarbon type solvent or silicone type solvent or a mixture thereof.

The amount of the water-soluble polymerization initiator is preferably 0.03 to 5% by weight, more preferably 0.1 to 2% by weight, based on the amount of the hydrophobic vinyl monomer. The polymerization initiator is previously mixed with other components and used as a solution, and for the purpose of reducing the remaining monomer, it may be diluted with a solvent etc. during polymerization and added all at once or continuously. The polymerization temperature is preferably 20 to 120°C, and the polymerization time is preferably 20 to 180 minutes.

The surface-hydrophobated water-absorbing polymer particles used in the present invention are preferably those obtained by polymerization of a hydrophilic vinyl monomer and a crosslinking agent in the presence of a silicone compound having at least one kind of functional group or a fluorine-based surfactant by a reverse phase suspension polymerization method, those obtained by heating water-absorbing polymer particles, a silicone compound having at least one kind of functional group, a fluorine-based surfactant or a silane coupling agent, in the presence of water, and those obtained by reverse phase suspension polymerization of a hydrophilic vinyl monomer and a crosslinking agent in the presence of a water-soluble polymerization initiator and a dispersant, and during or after polymerization, adding a hydrophobic vinyl monomer and an oil-soluble polymerization initiator, followed by polymerization thereof.

### [Anti-perspiring component]

Preferable examples of the anti-perspiring component include aluminum compounds, zirconium compounds, zinc compounds and mixtures thereof. Preferable examples include their sulfates, bromides, chlorides, chlorohydroxides, lactates etc., specifically aluminum salts such as allantoin chlorohydroxy aluminum, aluminum chloride, chlorohydroxy aluminum, allantoin dihydroxy aluminum, aluminum sulfate, potassium alum and aluminum hydroxide, aluminum-zirconium complexes such as aluminum zirconium tetrachlorohydroxy glycine and aluminum zirconium pentachlorohydrate, and zinc salts such as zinc oxide and zinc p-phenol sulfonate. Among these, the aluminum compounds and zirconium compounds are preferable, the aluminum salts and aluminum-zirconium complexes are more preferable, and the aluminum-zirconium complexes are particularly preferable.

### [Silicone]

Preferably, the cosmetic composition of the present invention further contains silicone to improve smoothness during application and shaving. As the viscosity (25°C) of such silicone is increased by using it in a larger amount, oily feeling is caused, so that in respect of feeling in use, the viscosity is preferably not higher than 5 × 10⁻³ m²/s, more preferably not higher than 5 × 10⁻⁴ m²/s, still more preferably not higher than 5 × 10⁻⁵ m²/s and even more preferably not higher than 2 × 10⁻⁵ m²/s. The lower limit is preferably not lower than 1 × 10⁻⁶ m²/s.

Specific examples of silicone include linear silicones such as low-polymerized dimethyl polysiloxane and cyclic silicones such as octamethyl cyclotetrasiloxane, decamethyl cyclopentasiloxane etc., and one or more of these silicones can be suitably selected and used.

### [Hydrophobic particles]

The cosmetic composition of the present invention preferably contains hydrophobic particles to further improve smoothness upon shaving and dry feeling on the skin.

Preferable examples of the hydrophobic particles include fine polymer particles obtained by suspension polymerization of a vinyl monomer in a solvent with a polysiloxane compound having a radical-polymerizable group at one end as a dispersant, that is, silicone-based resin such as silicone resin (KMP-590 (manufactured by Shin-Etsu Chemical Co., Ltd.)), Tospearl 145, Tospearl 2000B (manufactured by GE Toshiba Silicones Co., Ltd.), Torefil (manufactured by Toray Industries, Inc.) etc.; fluorine-based resin; particles obtained by subjecting organic powder such as nylon resin (SP-500 (manufactured by Toray Industries, Inc.) etc.), polystyrene-based resin (Fine Pearl (manufactured by Sumitomo Chemical Co., Ltd.), Techno Polymer SB (manufactured by Sekisui Plastics Co., Ltd.), Fine Powder SGP (manufactured by Soken Chemical & Engineering Co., Ltd.) etc.), polyethylene resin (Flow Beads (manufactured by Sumitomo Seika Co., Ltd.) etc.), polymethyl methacrylate-based resin (Matsumoto Microsphere M (manufactured by Matsumoto Oil and Fat Co. , Ltd.), Tech Polymer MB (manufactured by Sekisui Plastics Co., Ltd.), Fine Powder MP (manufactured by Soken Chemical & Engineering Co., Ltd.) etc.), divinyl benzene-based resin, polyurethane-based resin, benzoguanamine resin, melamine resin, or phenol-based resin, to hydrophobation treatment such as silicone treatment (for example, treatment with methyl hydrogen polysiloxane or dimethyl polysiloxane), fluorine treatment, metallic soap treatment or fatty acid treatment, preferably silicone treatment or fluorine treatment; and particles obtained by subj ecting inorganic powder such as talc, sericite, mica, kaolin, red oxide, clay, bentonite, silicic anhydride, biotite or synthetic silica beads, to hydrophobation treatment such as silicone treatment (for example, treatment with methyl hydrogen polysiloxane or dimethyl polysiloxane), fluorine treatment, metallic soap treatment or fatty acid treatment, preferably silicone treatment or fluorine treatment.

The shape of the hydrophobic particles may be in the form of sphere, tube, plate or needle, preferably in the form of sphere in respect of smoothness. The roundness of the hydrophobic particles is not particularly limited, but as the roundness is increased, the dynamic coefficient of friction is decreased to improve smoothness on the skin, and thus hydrophobic particles that are as round as possible are preferably used. The average particle diameter of the hydrophobic particles is preferably 0.05 to 50 µm, particularly preferably 0.5 to 50 µm.

### [Cosmetic composition]

The cosmetic composition of the present invention may be any of emulsified cosmetics, oil cosmetics, spray cosmetics, stick-type cosmetics, powdered cosmetics and roll-on type cosmetics, and is used preferably for antiperspiration.

The cosmetic composition of the present invention contains water-absorbing polymer particles, preferably surface-hydrophobated water-absorbing polymer particles. The content (content in a state not swollen with water) of the water-absorbing polymer particles in the composition is preferably 0.1 to 90% by weight, more preferably 0.5 to 50% by weight, still more preferably 1 to 20% by weight and even more preferably 2 to 10% by weight. The polymer particles in this range are preferable because they can suppress stickiness after absorption of sweat. Further, the surface-hydrophobated water-absorbing polymer particles are used preferably as water-absorbing polymer particles in order to maintain the dry feel of the particles after absorption of sweat to enable smooth shaving.

The content of the anti-perspiring component in the cosmetic composition of the present invention is preferably 0.1 to 90% by weight, more preferably 1 to 70% by weight, still more preferably 5 to 50% by weight and even more preferably 10 to 30% by weight. The polymer particles in this range are preferable because of their high anti-perspiring effect.

The amount of the water-absorbing polymer particles is preferably 3 to 95 parts by weight, more preferably 3 to 50 parts by weight, based on 100 parts by weight of the anti-perspiring component. The particles in an amount in this range are preferable because the deterioration in smoothness upon shaving caused by precipitation of an aluminum salt can be solved by simultaneously using the water-absorbing polymer particles in the above range, and both the components can be maintained at suitable pH value at the time of perspiring.

The content of silicone having a viscosity (25°C) of not higher than 5 × 10⁻³ m²/s in the cosmetic composition of the present invention is preferably 0.1 to 90% by weight, more preferably 0.5 to 50% by weight, still more preferably 1 to 20% by weight and even more preferably 2 to 10% by weight.

The content of the hydrophobic particles in the cosmetic composition of the present invention is preferably 0.5 to 30% by weight, more preferably 0.5 to 20% by weight, still more preferably 0.5 to 10% by weight and even more preferably 1 to 5% by weight.

Particularly, when usual water-absorbing polymer particles whose surface is not hydrophobated are used, at least one member selected from the group consisting of silicone having a viscosity (25°C) of not higher than 5 × 10⁻³ m²/s and hydrophobic particles is preferably used in order to improve smoothness in shaving.

Depending on the form, type etc. of the cosmetic composition of the present invention, other generally used cosmetic components can be further incorporated into the cosmetic composition in such a range that the effect of the present invention is not hindered.

Such cosmetic components include e.g. extender pigments such as mica, talc, sericite, kaolin, polymethylsilyl sesquioxane and barium sulfate; inorganic pigments such as titanium oxide, zinc white and iron oxide; hydrocarbons such as solid or liquid paraffin, solid paraffin, microcrystalline wax, vaseline, ceresin, ozokerite and montan wax; vegetable or animal fats and oils or wax, such as olive, ozokerite, carnauba wax, lanoline and spermaceti; fatty acids or esters thereof such as stearic acid, palmitic acid, oleic acid, glycerine monostearate, glycerine distearate, glycerine monooleate, isopropyl myristate, isopropyl stearate and butyl stearate; higher alcohols such as cetyl alcohol, stearyl alcohol, palmityl alcohol and hexyl dodecyl alcohol; adsorbents or thickening agents such as cationic cellulose, carboxybetaine type polymer and cationic silicone; polyhydric alcohols having a moisture retention action, such as glycol and sorbitol; efficacious components such as whitening agent, analgesic antiinflammatory agents, anti-itching agents, astringents, skin softening agents and hormones; water; surfactants; W/O or O/W type emulsifying agents; emulsifying agents for silicone oil, such as polyether-modified silicone, polyether alkyl-modified silicone and glyceryl ether-modified silicone; thickening agents such as methyl cellulose, ethyl cellulose, carboxymethyl cellulose, polyacrylic acid, tragacanth, agar and gelatin; lower alcohols such as ethanol, glycerin, 1,3-butylene glycol and propylene glycol; and other components such as emulsion stabilizers, chelating agents, UV protecting agents, pH adjusting agents, preservatives, coloring matters and perfumes.

The cosmetics of the present invention are produced according to a conventional method.

The cosmetic composition of the present invention exhibits a high anti-perspiring effect to enable further smooth shaving. Particularly, the surface-hydrophobated water-absorbing polymer particles can be used to give highly dry feel even after perspiring.

### Examples

In the following examples, "%" refers to "% by weight" unless otherwise specified.

### Synthesis Example 1

300 g methacrylic acid (Mitsubishi Rayon Co., Ltd.) and 135 g ion-exchange water were placed in a 3-L beaker and then neutralized to a degree of 75 % by adding 348 g of 30% aqueous sodium hydroxide solution dropwise thereto under cooling with stirring, and then a solution of 1.2 g potassium persulfate (0.4% relative to methacrylic acid) dissolved in 24.3 g ion-exchange water and a crosslinking agent ethylene glycol diglycidyl ether (trade name, Denacol EX810, Nagase Kasei Co., Ltd.), 15.0 g (5.0% relative to methacrylic acid), were added thereto, and the mixture was uniformly dissolved. The resultant solution was added to a solution obtained by dissolving 15 g amino-modified silicone A (XF42-703 produced by GE Toshiba Silicones Co., Ltd.; viscosity (25°C), 1 × 10⁻³ m²/s; amine equivalent, 1500 g/mol) in 1500 ml cyclohexane in a 3-L beaker, and the mixture was stirred vigorously for 5 minutes at a number of revolutions of 10000 rpm in a homomixer to produce a fine water-in-oil droplet dispersion. Then, 900 ml cyclohexane was introduced into a 5-L stainless steel reaction vessel equipped with a reflux condenser, then stirred at 350 rpm, and heated at 75°C in the system, and the atmosphere was replaced by nitrogen, followed by dropwise addition of the partially neutralized water-in-oil droplet dispersion of methacrylic acid to initiate polymerization. The whole of the droplet dispersion was added dropwise over 1.5 hours, and the reaction solution was aged for additional 4 hours at the reflux temperature. Thereafter, a dehydration tube was attached, the temperature was raised to remove 340 ml water, and the reaction solution was cooled and filtered to separate polymer particles. By evaporation into dryness under reduced pressure, 376 g dry white polymer in the form of fine powder was obtained. The average particle diameter of the resultant fine polymer particles was 2.2 µm, and the amount of water absorbed was 15 g/g.

### Synthesis Example 2

180 g aqueous solution of a neutralized product (effective component, 90%) of dimethylaminoethyl methacrylate with diethylsulfuric acid, 120 g N,N-dimethylacrylamide, 14 g polyethylene glycol dimethacrylate (trade name: NK-14G, manufactured by ShinNakamura Chemical Co., Ltd.), 5 g methacrylic acid, 415 g ion-exchange water and 1.2 g potassium persulfate were introduced into a 3-L beaker and dissolved uniformly. The obtained solution was added to a solution obtained by dissolving 15 g amino-modified silicone A (XF42-703 produced by GE Toshiba Silicones Co., Ltd.; viscosity (25°C), 1 × 10⁻³ m²/s; amine equivalent, 1500 g/mol) in 1500 ml cyclohexane in a 3-L beaker. The mixture was stirred vigorously for 5 minutes at a number of revolutions of 10000 rpm in a homomixer to produce a fine water-in-oil droplet dispersion. The water-in-oil droplet dispersion was introduced into a 5-L stainless steel reaction vessel equipped with a reflux condenser and then stirred at 350 rpm. Purged by nitrogen gas, it was heated at 75°C and it was kept for 4 hours for polymerization. Thereafter, a dehydration tube was attached, the temperature was raised to remove 340 ml water and the reaction solution was cooled and filtered to separate polymer particles. By evaporation into dryness at a reduced pressure, 280 g dry white polymer in the form of fine powder was obtained. The average particle diameter of the resultant fine polymer particles was 2.5 µm, and the amount of water absorbed was 18 g/g.

### Synthesis Example 3

300 g N,N-dimethylacrylamide, 4.5 g polyethylene glycol dimethacrylate (trade name: NK-14G, manufactured ShinNakamura Chemical Co., Ltd.), 5 g methacrylic acid, 400 g ion-exchange water and 1.2 g potassium persulfate were introduced into a 3-L beaker and dissolved uniformly. The obtained solution was added to a solution obtained by dissolving 15 g amino-modified silicone A (XF42-703 produced by GE Toshiba Silicones Co., Ltd.; viscosity (25°C), 1 × 10⁻³ m²/s; amine equivalent, 1500 g/mol) in 1500 ml of cyclohexane in a 3-L beaker. The mixture was stirred vigorously for 5 minutes at a number of revolutions of 10000 rpm in a homomixer to produce a fine water-in-oil droplet dispersion. The water-in-oil droplet dispersion was introduced into a 5-L stainless steel reaction vessel equipped with a reflux condenser and then stirred at 350 rpm. Purged by nitrogen gas, it was heated at 75°C and it was kept for 4 hours for polymerization. Thereafter, a dehydration tube was attached, the temperature was raised to remove 340 ml water and the reaction solution was cooled and filtered to separate polymer particles. By evaporation into dryness at a reduced pressure, 290 g dry white polymer in the form of fine powder was obtained. The average particle diameter of the resultant fine polymer particles was 2.3 µm, and the amount of water absorbed was 18 g/g.

### Synthesis Example 4

300 g methacrylic acid (Mitsubishi Rayon Co., Ltd.) and 135 g ion-exchange water were placed in a 3-L beaker and neutralized to a degree of 75% by adding 348 g of 30% aqueous sodium hydroxide solution dropwise thereto under cooling with stirring, and then a solution of 1.2 g potassium persulfate (0.4% relative to methacrylic acid) dissolved in 24.3 g ion-exchange water, and 15. 0 g of a crosslinking agent ethylene glycol diglycidyl ether (trade name, Denacol EX810, Nagase Kasei Co., Ltd.) (5.0% relative to methacrylic acid), were added thereto, and the mixture was uniformly dissolved. The resultant solution was added to a solution obtained by dissolving 15 g sugar esters (a mixture of Ryoto Sugar Esters S570 and S770 (trade name) in equal amounts, Mitsubishi Shokuhin Co. , Ltd.) (5.0% relative to methacrylic acid) in 1500 ml cyclohexane in a 3-L beaker, and the mixture was stirred vigorously for 5 minutes at a number of revolutions of 10000 rpm in a homomixer to produce a fine water-in-oil droplet dispersion. Then, 900 ml cyclohexane was introduced into a 5-L stainless steel reaction vessel equipped with a reflux condenser, then stirred at 350 rpm, and heated at 75°C in the system, and the atmosphere was replaced by nitrogen, followed by dropwise addition of the partially neutralized water-in-oil droplet dispersion of methacrylic acid to initiate polymerization. The whole of the droplet dispersion was added dropwise over 1.5 hours, and the reaction solution was aged for additional 4 hours at the reflux temperature. Thereafter, a dehydration tube was attached, the temperature was raised to remove 340 ml water, further 1300 ml cyclohexane was distilled away, and the reaction solution was left and cooled to complete synthesis. By evaporation into dryness under reduced pressure, 376 g dry white polymer in the form of fine powder was obtained. The average particle diameter of the resultant fine polymer particles was 2.2 µm, and the amount of water absorbed was 12 g/g.

### Examples 1 to 4 and Comparative Example 1

The water-absorbing polymer particles obtained in each of Synthesis Examples 1 to 4 were mixed uniformly under heating with all the components shown in Table 1, and the resultant mixture was introduced into a container, then left and cooled to give a stick-type antiperspirant. The resultant antiperspirant was evaluated for anti-perspiring performance and smoothness and stress upon shaving. The results are collectively shown in Table 1.

### <Method of evaluating anti-perspiring performance>

The test sample, 0.3 g, was applied onto one armpit, and 3 hours later, an examinee was allowed to be in a high-temperature high-humidity room (40°C/80% RH) for 5 minutes, and just after leaving the room, the examinee herself evaluated anti-perspiring performance sensorily when perspired. Subsequently, the degree of wetting on the armpit 3 hours after leaving the room was evaluated sensorily as anti-perspiring performance in a normal state by the examinee herself under the conditions of 25°C/60% RH. The anti-perspiring performance was evaluated in 5 ranks under the following criteria.
1: Evidently wet.
2: Wet.
3: Slightly wet.
4: Dry.
5: Evidently dry.

### <Method of evaluating smoothness upon shaving>

Five hours after the above evaluation of anti-perspiring performance was finished, lather foamed from soap in a bathroom was applied onto the armpit, and the armpit was shaved with a commercial razor, and smoothness upon shaving was evaluated sensorily by the examinee herself in 5 ranks under the following criteria:
1: Very poor in smoothness.
2: Poor in smoothness.
3: Slightly poor in smoothness.
4: Excellent in smoothness.
5: Very excellent in smoothness.

### <Measurement of stress upon shaving>

0.3g of the test sample was applied onto one forearm, and soap made in the US (DAVE manufactured by Unilever) was applied onto the forearm, and the armpit was shaved with a commercial razor equipped with a stress meter (Handy Force Gauge, manufactured by Nippon Keisoku System Co., Ltd.), and the maximum stress (N) during shaving was measured. The measurement was conducted 6 times by each of 3 examinees, and the average value was indicated as stress (N) upon shaving.

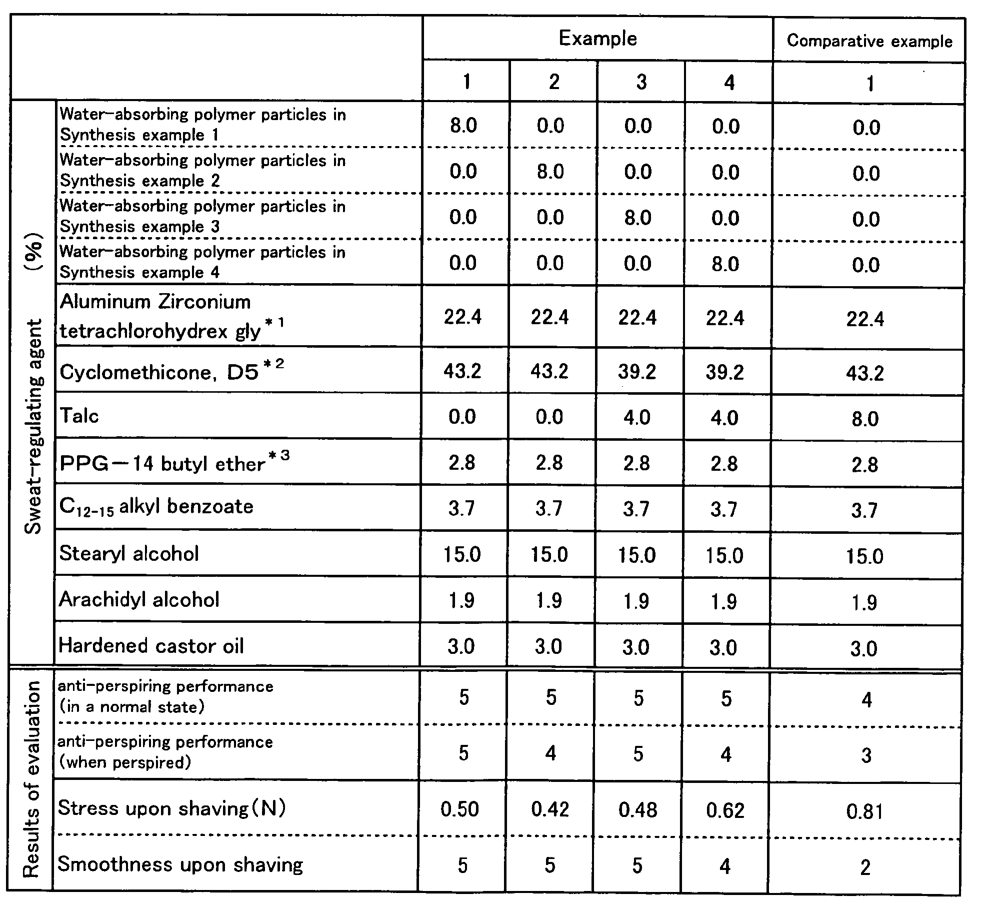

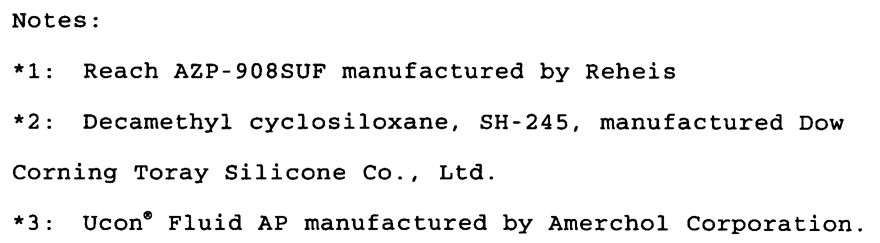

Hereinafter, formulation examples are shown.

### Formulation Example 1: Powdered antiperspirant

| <Composition> | |
|---|---|
| a) Aluminum zirconium pentachlorohydrate | 15.0% |
| b) Trichlosane | 0.01 |
| c) Water-absorbing polymer particles in Synthesis Example 1, | 5.0 |
| d) Talc | balance |
| e) Perfume | suitable amount |

### Formulation Example 2: Aerosol type antiperspirant

| <Composition> | |
|---|---|
| a) Aluminum zirconium pentachlorohydrate | 8.0% |
| b) Water-absorbing polymer particles in Synthesis Example | 1 2.0 |
| c) Acrylic polymer particles* | 1.0 |
| d) Talc | 0.5 |
| e) Isopropyl palmitate | 1.5 |
| f) Perfume | 0.2 |
| g) Isopentane | 10.0 |
| h) Liquefied petroleum gas | balance |
| *: Matsumoto Microsphere M305 (particle diameter 7 µm) manufactured by Matsumoto Oil and Fat Co., Ltd. | |

### Formulation Example 3: Non-aqueous roll-on type antiperspirant

| <Composition> | |
|---|---|
| a) Octamethyl cyclotetrasiloxane | 67.0% |
| b) Dimethicone 6cs* | 5.0 |
| c) Ethanol | 5.0 |
| d) Water-absorbing polymer particles in Synthesis Example 1 | 3.0 |
| e) Aluminum zirconium pentachlorohydrate | 20.0 |
| *: KF96-6cs manufactured by Shin-Etsu Chemical Co., Ltd. | |

### Formulation Example 4: Soft solid type antiperspirant

| <Composition> | |
|---|---|
| a) Octamethyl cyclotetrasiloxane | 54.0% |
| b) Behenyl alcohol | 6.0 |
| c) Mineral oil*¹ | 7.0 |
| d) Water-absorbing polymer particles in Synthesis Example 1 | 3.0 |
| e) Aluminum zirconium pentachlorohydrate | 20.0 |
| f) Alkyl-modified silicone*² | 2.0 |
| g) Cyclomethicone/dimethiconecross polymer*³ | 1.5 |
| h) Colloidal silica | 2.5 |
| i) Talc | 4.0 |

| | |
|---|---|
| *1: DRAKEOL® LT MINERAL OIL manufactured by N.F. Penreco | |
| *2: TSF4421 manufactured by GE Toshiba Silicones Co., Ltd. | |
| *3: KSG16 manufactured by Shin-Etsu Chemical Co., Ltd. | |

## Claims

1. A cosmetic composition comprising surface-hydrophobated water-absorbing polymer particles and an anti-perspiring component.

2. The cosmetic composition according to claim 1, wherein the surface-hydrophobated water-absorbing polymer particles comprise water-absorbing polymer particles coated thereon with a silicone compound having at least one kind of functional group.

3. A cosmetic composition comprising water-absorbing polymer particles, an anti-perspiring component, and at least one member selected from the group consisting of silicone having a viscosity (25°C) of not higher than 5 × 10⁻³ m²/s and hydrophobic particles, the content of the water-absorbing polymer particles being 3 to 95 parts by weight relative to 100 parts by weight of the anti-perspiring component.

4. The cosmetic composition according to any one of claims 1 to 3, wherein the average particle diameter of polymer particles selected from the water-absorbing polymer particles and the surface-hydrophobated water-absorbing polymer particles is 0.1 to 50 µm.

5. The cosmetic composition according to any one of claims 1 to 4, wherein the amount of water absorbed into the polymer particles selected from the water-absorbing polymer particles and the surface-hydrophobated water-absorbing polymer particles is 5 to 100 g/g.

6. The cosmetic composition according to any one of claim 1 to 5, wherein the anti-perspiring component is at least one member selected from the group consisting of an aluminum compound, a zirconium compound and a zinc compound.

7. Use of a composition comprising surface-hydrophobated water-absorbing polymer particles and an anti-perspiring component as cosmetics.

8. A method of antiperspiration, comprising applying a composition comprising surface-hydrophobated water-absorbing polymer particles and an anti-perspiring component onto the skin.
